# EUROPEAN PATENT SPECIFICATION

(11) **EP 0 197 592 B1**
(45) Date of publication and mention of the grant of the patent: **17.02.1993**
(21) Application number: 86200518.8
(22) Date of filing: 26.03.1986
(51) Int. Cl.: C12N 15/00, C12N 1/20, C12P 21/02, A61K 37/02, A61K 35/16

(54) **Preparation of the human von Willebrand factor by recombinant DNA**
Herstellung des menschlichen von-Willebrand-Faktors durch rekombinante DNS
Préparation du facteur von Willebrand humain par un ADN recombinant

(30) Priority: 01.04.1985 NL 8500961
(43) Date of publication of application: 15.10.1986
(73) Proprietor: STICHTING VRIENDEN VAN DE STICHTING DR. KARL LANDSTEINER, 1066 CX Amsterdam (NL)
(72) Inventor: Pannekoek, Hans, NL-1431 EW Aalsmeer (NL); Verwey, Cornelis Lammert, NL-1391 GN Abcoude (NL); Diergaarde, Paul Johan, NL-1111 ZX Diemen (NL); Hart, Margaretha Hendrika L., NL-1061 GW Amsterdam (NL)
(74) Representative: Davies, Jonathan Mark

(56) References cited:
- GB-A- 2 079 292
- EXPERIENTIA, vol. 39, no. 6, June 1983, page 656, Birkhauser Verlag, Basel, CH; B. WYLER et al.: "Fragments of bovine von Willebrand factor which influence platelet aggregation by the intact factor"
- NATURE, vol. 312, no. 5992, November 1984, pages 330-336, Reading, Berks., GB; W. WOOD et al.: "Expression of active human factor VIII from recombinant DNA clones"
- NATURE, vol. 312, no. 5992, November 1984, pages 342-347, Reading, Berks., GB; J. TOOLE et al.: "Molecular cloning of a cDNA encoding human antihaemophilic factor"
- PROC. NATL. ACAD. SCI., vol. 82, October 1985, pages 6394-6398, US; J. SADLER et al.: "Cloning and characterization of two cDNAs coding for human von Willebrand factor"
- SCIENCE, vol. 228, no. 4706, 21st June 1985, pages 1401-1406; D. GINSBURG et al.: "Human von Willebrand factor (vWF): isolation of cemplementary DNA (cDNA) clones and chromosomal localization"
- CELL, vol. 41, May 1985, pages 49-56; D. LYNCH et al.: "Molecular cloning of cDNA for human von Willebrand factor: authentication by a new method"
- NUCLEIC ACIDS RESEARCH, vol. 13, no. 13, 1985, pages 4699-4717, IRL Press Limited, Oxford, GB; C. VERWEY et al.: "Construction of cDNA coding for human von Willebrand factor using antibody probes for colony-screening and mapping of the chromosomal gene"

## Description

cDNA coding for the human von Willebrand factor, plasmids or phages with such a cDNA coding or fragments thereof respectively, microorganisms and animal or human cells which contain such plasmids or phages, preparation of proteins by the cultivation of the above mentioned hosts, the obtained proteins and pharmaceutical compositions containing a biologically active form of the obtained proteins.

The von Willebrand factor (vWF) is a large, multimeric plasma protein, composed of an apparently, single glycoprotein with a molecular weight (MW) of about 225 kD. These subunits are linked together by disulfide bonds. In plasma vWF circulates as multimers, ranging from dimers to multimers of more than 50 subunits. Dimers consist of two subunits joined, probably at their C-termini, by flexible "rod-shaped" domains and are presumed to be the protomers in multimerization. The protomers are linked through large, probably N-terminal, globular domains to form multimers.

vWF is synthesized by endothelial cells and megakaryocytes. It is believed that this protein is initially produced as a 260 kD glycosylated precursor that is subsequently subjected to carbohydrate processing, sulfatation, dimerization, multimerization and to proteolytic cleavage to yield the mature 225 kD subunit (Sporn, L.A., et al. (1985) Biosynthesis of vWF protein in human megakaryocytes, J. Clin. Invest. 76, 1102-1106); Wagner, D.D., et al. (1984) J. of Cell Biology 99, 2123-2130). It has been shown that vWF is stored in the Weibel-Palade bodies within the endothelial cells. It can not be excluded that these organelles play a role in the processing of the precursor protein.

vWF participates in critical steps in hemostasis. It is involved in platelet-vessel wall interactions after vascular injury, leading to platelet plug formation. On the vWF protein domains have been assigned which show specific interaction with the platelet glycoproteins IB (Jenkins, C.S.P., et al. (1976) J. Clin. Invest. 69, 1212-1222), IIB/IIIA (Fujimoto, T., et al. (1982) Adenomie diphosphate induces binding of von Willebrand factor to human platelets, Nature 297, 154-156), collagens type I and III and with another, yet unidentified, component in the subendothelium. These assignments are based on studies with monoclonal anti-vWF antibodies which are able to inhibit a particular interaction of vWF. For a in depth analysis of structure-function relationships of the vWF protein, a full length vWF cDNA will be indispensable. Introduction of well-defined mutations within this cDNA and expression of the mutated cDNA in a suitable host will allow a detailed localization of functional domains within the vWF protein. Recently, applicants and others (Lynch, D.C., et al. (1985) Cell 41, 49-56; Ginsburg, M., et al. (1985) J. Biol. Chem. 260, 3931-3936; Verweij, C.L., et al. (1985) Nucleic Acids Research 13, 4699-4717, based on Dutch patent application 85.00961; Sadler, J.E., et al. (1985) Proc. Natl. Acad. Sci. USA 82, 6394-6398) have cloned partial vWF cDNA sequences. The presence of a short 3' untranslated region (136 nt) on vWF mRNA, which extends to about 9,000 nt, led us to assume that the precursor protein for vWF has a MW considerable larger than the reported 260 kD (Wagner, D.D., et al. (1984) J. of Cell Biology 99, 2123-2130). A full length vWF cDNA will enable us to elucidate the enigma on the MW of the precursor, characterize its processing pathway and establish the primary structure.

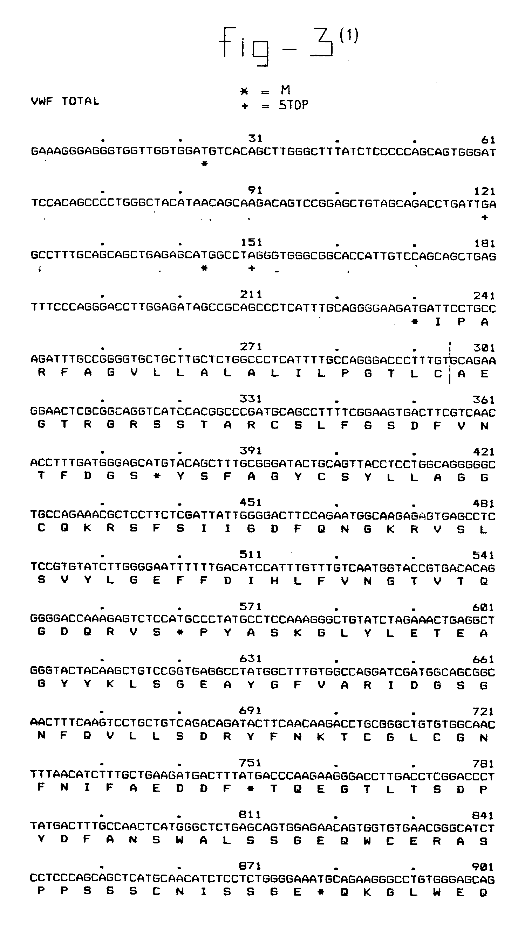

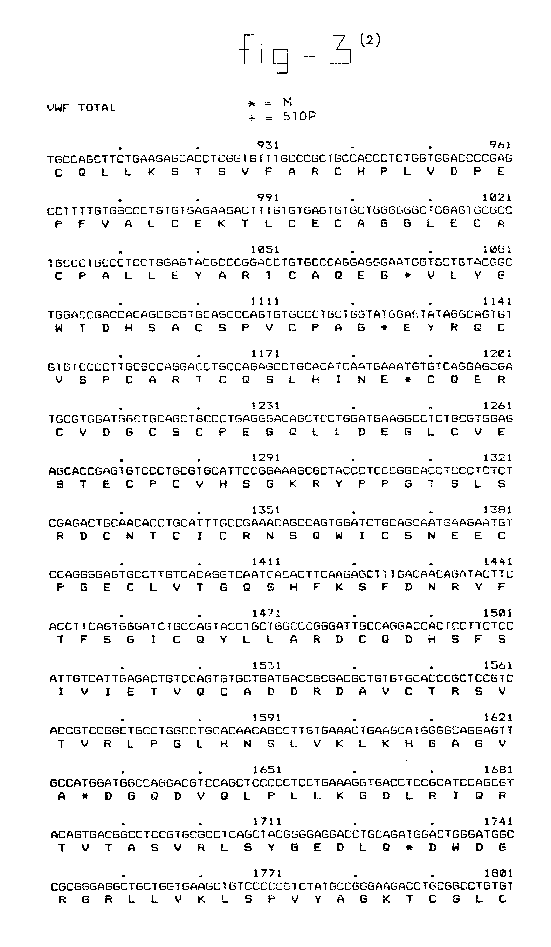

This invention relates to recombinant DNA, comprising a DNA fragment having a base sequence essentially as shown by nucleotides 229-8670 in figure 3 which codes for a full-length human von Willebrand factor precursor. Said recombinant DNA may further comprise a DNA fragment derived from a vector plasmid or phage.

The invention also provides a human or animal cell or microorganism which, as a result of genetic engineering, contains such recombinant DNA. Preferably, said cell is capable of producing biologically active human von Willebrand factor.

In addition, this invention provides a method for the preparation of biologically active human von Willebrand factor, comprising cultivating such a cell which is capable of producing biologically active human von Willebrand factor and isolating the biologically active human von Willebrand factor produced.

This invention also provides a method for the preparation of a pharmaceutical composition comprising biologically active human von Willebrand factor, comprising cultivating such a cell which is capable of producing biologically active human von Willebrand factor, isolating and purifying the biologically active human von Willebrand factor produced, and formulating it together with a pharmaceutically acceptable carrier or excipient into a pharmaceutical composition. In this respect it is emphasized that by means of such pharmaceutical compositions it is possible to circumvent the safety problems bound to the administration of vWF protein recovered from blood samples of normal individuals.

To perform the investigations forming the basis of the invention, use has been made of the materials and method below

### MATERIALS AND METHODS

### cDNA cloning:

Total RNA was purified from in vitro cultured endothelial cells, derivated from veins of human umbilical cords (Verweij, C.L., et al. (1985) Nucleic Acids Research 13, 4699-4717, based on Dutch patent application 85.00961). Primer-directed cDNA was synthesized essentially according to a protocol described (Gubler, U., et al. (1983) Gene 25, 263-269; Toole, J.J., et al. (1985) Nature 312, 342- 347). The cDNA synthesis was arrested by adding EDTA and SDS till a final concentration of, respectively, 20 mM and 0.1 %. The cDNA preparations were extracted with phenol-chloroform, then precipitated with ethanol and purified by chromatography on Sephadex G-50. In the case of primer-directed cDNA synthesis with primer A (5' CACAGGCCACACGTGGGAGC 3'), complementary to nucleotides 6901 till 6921, the cDNA preparation was digested with BglII (positions 6836 and 2141) and KpnI (position 4748). Subsequently, the digested cDNA was size-fractionated by chromatography on a Sepharose CL-4B column. Fractions containing cDNA larger than about 600 bp were ligated to plasmid pMBLll, digested with BglII and KpnI. A cDNA library of about 15,000 independent colonies was established, using strain E.coli DHl as a host, which was screened with two oligonucleotide probes (B and C). Probe B (5' GAGGCAGGATTTCCGGTGAC 3'), complementary to nucleotides 4819 till 4839 was employed for the isolation of the plasmid pvWF2084, harboring a 2,084 bp BglII-KpnI vWF cDNA fragment, while probe C (5' CAGGGACACCTTTCCAGGGC 3'), complementary to 2467 till 2487, was used for the detection of plasmid pvWF2600, harboring an approximately 2,600 bp KpnI-BglII vWF cDNA fragment.

Using probe C for primer-directed synthesis applicants divided the resulting cDNA preparation in two parts. One part was C-tailed and annealed to G-tailed plasmid pUC9 as described before (Verweij, C.L., et al. (1985) Nucleic Acids Research 13, 4699-4717, based on Dutch patent application 85.00961) and used to transform E.coli DHl. Six thousand independent colonies were hybridized with a "nick translated" 575 bp BglII-BamHI vWF cDNA fragment of pvWF2600 DNA. A positive clone, harboring a plasmid with the longest insert (about 1,800 bp, designated pvWF1800) was chosen for further study. The other part of the primer C-directed cDNA preparation was treated with EcoRI methylase and subsequently with T4-DNA polymerase and dNTPs to ensure blunt-ended termini (Maniatis, T., et al. (1982) Molecular Cloning Laboratory Manual, Cold Spring Harbor Laboratory). Phosphorylated EcoRI linkers (New England Biolabs, Beverly, MA) were ligated to the termini of the cDNA preparation and unreacted components were removed by Sephadex G-50 chromatography. The XhoI site, located about 350 bp downstream of the 5' end of the vWF cDNA insert of pvWF1800 DNA, was used for another selection. After digestion with an excess of EcoRI and XhoI chromatography on Sepharose CL-4B was employed to remove digested EcoRI linkers. The final preparation was ligated to plasmid pMBLll DNA which had been digested with EcoRI plus XhoI and used to transform E.coli DHl. A collection of about 10,000 independent colonies was hybridized with a "nick translated" 350 bp XhoI-HindIII vWF cDNA fragment from plasmid pvWF1800. The HindIII site of this fragment has been derived from the polylinker of the vector pUC9. A positive clone, harboring the longest insert (about 1,330 bp, designated pvWF1330) was further studied.

### S1 nuclease protection analysis:

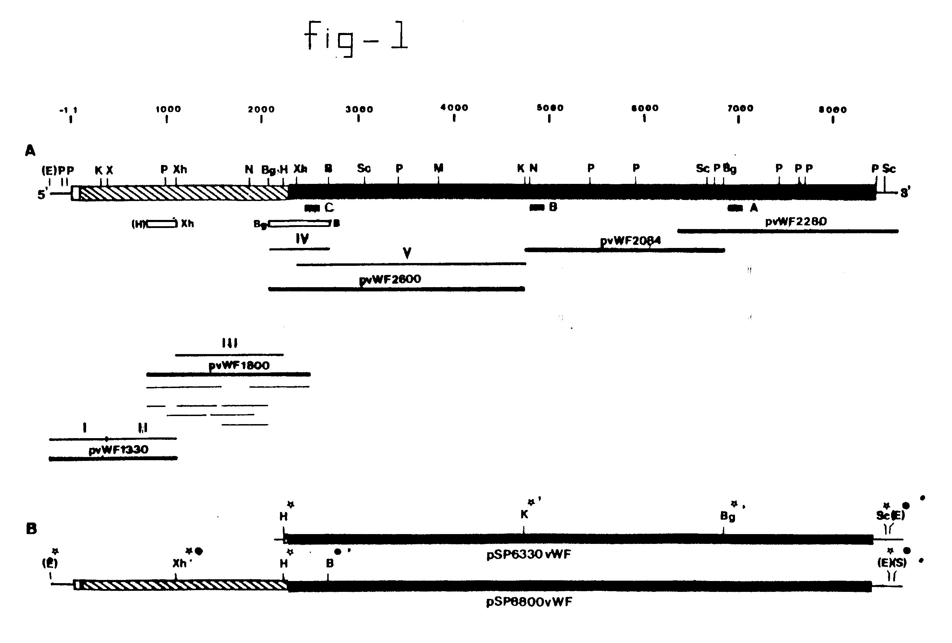

Applicants used a probe for S1 nuclease protection experiments an XhoI-EcoRI fragment of about 5,300 bp (probe V) from plasmid pvWF2600 which contains a 2,390 bp segment (XhoI-KpnI) constituted of vWF cDNA (Fragment V, Figure 1). Probe II was a 4,800 bp XbaI-EcoRI fragment from plasmid pvWF1330 which harbors a 735 bp XbaI-XhoI vWF cDNA segment (Fragment II, Figure 1). The fragments were 3' end labelled, using DNA polymerase I (large fragment) (New England Biolabs, Beverly, MA) to fill in recessed ends (Maniatis, T., et al. (1982) Molecular Cloning Laboratory Manual, Cold Spring Harbor Laboratory). Subsequently, these probes were isolated by electrophoresis on a 0.7 % low-melting agarose gel and purified as described (Wieslander, L. (1979) Anal. Biochem. 48, 305-309).

Three other vWF cDNA fragments were subcloned in double stranded M13mp18 and employed as probes. To that end the anti-sense DNA strand was uniformily labelled by elongation from the universal M13-primer with DNA polymerase I (large fragment). The subcloned fragments were a 1,144 bp XhoI- HindIII fragment of plasmid pwF1800 (Fragment III, Figure 1), a 585 bp XbaI-EcoRI fragment of plasmid pvWF1330 (Fragment I, Figure 1) and a 575 bp BamHI-BglII fragment of plasmid pvWF2600 (Fragment IV, Figure 1). After DNA synthesis, initiated at the M13 primer, double stranded DNA was digested with both HindIII and PvuII for fragment III (to yield probe II), with both XbaI and EcoRI for fragment I (to yield probe I) and with both BamHI and PvuII for fragment IV (to yield probe IV). The rationale for the construction of probes II, III, IV and V is that they contain a segment of vector DNA non-complementary with endothelial RNA. For example probes III and IV harbor about 200 bp, derived from M13mp18. These probes were subjected to electrophoresis on a 5 % polyacrylamide - 8M urea gel and the fragments of interest were isolated (Maxam A.G., et al. (1980) Methods Enzymol. 65, 499-560).

S1 nuclease protection experiments were carried out essentially as described (Berk, A.J., et al. (1977) Cell 12, 721-732). One microgram of human endothelial polyA RNA was added to 10,000 - 100,000 counts per min. of radiolabelled probe, heated for 10 min. at 80°C, followed by an incubation overnight at 60°C for probes I, II, III and V and at 57°C for probe IV. Digestion with 200 Units of S1 nuclease (Bethesda Research Lab., Gaithersburg, Md.) per ml for 20 min. at 45°C. Undigested DNA was precipitated with ethanol and the pellets were dissolved in the appropriate loading buffer for electrophoresis on a 0.8 % alkaline agarose gel or on a 6 % polyacrylamide sequencing gel (Maniatis, T., et al. (1982) Molecular Cloning Laboratory Manual, Cold Spring Harbor Laboratory). The first procdedure was employed for probes I and III, whereas the second one was applied for probes II, IV and V.

### Assembly of full length vWF cDNA:

For the construction of plasmid pSP6330vWF, harboring a continuous vWF cDNA segment of about 6,331 bp extending from the HindIII site (position 2235) till the SacI site within the 3' untranslated region (position 8562) the following vWF cDNA fragments were isolated: the 2,517 bp HindIII-KpnI fragment (position 2236 till 4753) from pvWF2600 DNA, the 2,084 bp KpnI-BglII fragment (position 4753 till 6837) from pvWF2084 DNA and the 1,730 bp BglII-SacI fragment (position 6837 till 8567) from pvWF2280 DNA. These three vWF cDNA fragments were ligated simultaneously into the vector pSP64 (Melton, D.A., et al. (1984) Nucl. Acids Research 12, 7035-7056), digested with both HindIII and SacI. About half of the resulting transformants contained a plasmid (denoted pSP6330vWF) with the desired vWF cDNA insert of about 6,331 bp, as verified by restriction enzyme analysis.

For the construction of plasmid pSP8800vWF, harboring full length vWF cDNA, the following fragments were isolated: the 6,330 bp HindIII-EcoRI insert of plasmid pSP6330vWF (the EcoRI site is derived from the polylinker present on the vector), the 1,044 bp XhoI-HindIII fragment (position 1092 till 2236) from pvWF1800 and the 1,327 bp EcoRI-XhoI fragment (position - 236 till 1092) from pWF1330. A five-fold molar excess of each of these three fragments were again ligated simultaneously with vector pSP65 DNA, cleaved with EcoRI and treated with calf intestine alkaline phosphatase (Boehringer, Mannheim, BRD). About 30 % of the resulting colonies harbored a plasmid with the desired, full length vWF cDNA insert of about 8,795 bp, as verified by restriction enzyme analysis and nucleotide sequence analysis.

### In vitro transcription and translation:

In vitro transcription of linear SP6-based DNA templates with SP6 RNA polymerase (New England Nuclear, Dreieich, BRD) was performed in the presence of 0.1 mM UTP, CTP and ATP, 0.05 mM GTP and 2 mM of m7G(5')ppp(5')G (Pharmacia, Uppsala, Sweden) to provide mRNA preparations with a capped terminus (Melton, D.A., et al. (1984) Nucl. Acids Research 12, 7035-7056). In vitro translation of such 5' capped mRNAs was done in a rabbit reticulocyte lysate system (New England Nuclear, Dreieich, BRD), according to the manufacturer's specifications.

Analysis of the in vitro translation products was performed by electrophoresis on a 8 % SDS-polyacrylamide gel as described (Laemmli, U.K. (1970) Nature 227, 650-655).

### RESULTS

### Construction of partial vWF cDNA clones and assembly of full length vWF cDNA:

Previously, applicants and others have reported on the construction of plasmids containing part of a full length human vWF cDNA (Verweij, C.L., et al. (1985) Nucleic Acids Research 13, 4699-4717, based on Dutch patent application 85.00961; Lynch, D.C., et al. (1985) Cell 41, 49-56; Ginsburg, M., et al. (1985) J. Biol. Chem. 260, 3931-3936; Sadler, J.E., et al. (1985) Proc. Nucl. Acad. Sci. USA 82, 6394-6398). The most extended vWF cDNA, that applicants obtained from an oligo(dT)-primed human endothelial cDNA library, comprised about 2,280 bp (pvWF2280). Nucleotide sequence analysis revealed that this cDNA insert has been initiated at the polyA tail of vWF mRNA. To construct a full length vWF cDNA, applicants have isolated additional, overlapping vWF cDNA sequences which are located upstream of pvWF2280. For that purpose two biochemical selections were employed to enrich for the number of vWF cDNA harboring plasmids. First, oligonucleotide primers, derived from the partial nucleotide sequence (Sadler, J.E., et al. (1985) Proc. Nucl. Acad. Sci. USA 82, 6394-6398), were synthesized to direct cDNA synthesis with human endothelial polyA RNA as substrate. Second, cDNA preparations were digested with particular restriction endonucleases, known to dissect vWF cDNA at a limited number of sites (Ginsburg, M., et al. (1985) J. Biol. Chem. 260, 3931-3936; Sadler, J.E., et al. (1985) Proc. Nucl. Acad. Sci. USA 82, 6394-6398). These restriction sites can subsequently be employed to assemble a full length vWF cDNA. The cloning strategy is outlined in Figure 1A. The plasmids, containing adjacent vWF cDNA sequences, were designated pvWF1330, pvWF1800, pvWF2600, pvWF2084 and pvWF2280. The nucleotide sequence of the 5' part of pWF1330 DNA (corresponding with the 5' end of vWF mRNA) revealed that nonsense codons were present in all three reading frames. From this finding applicants conclude that pvWF1330 DNA extends beyond the translation initiation codon.

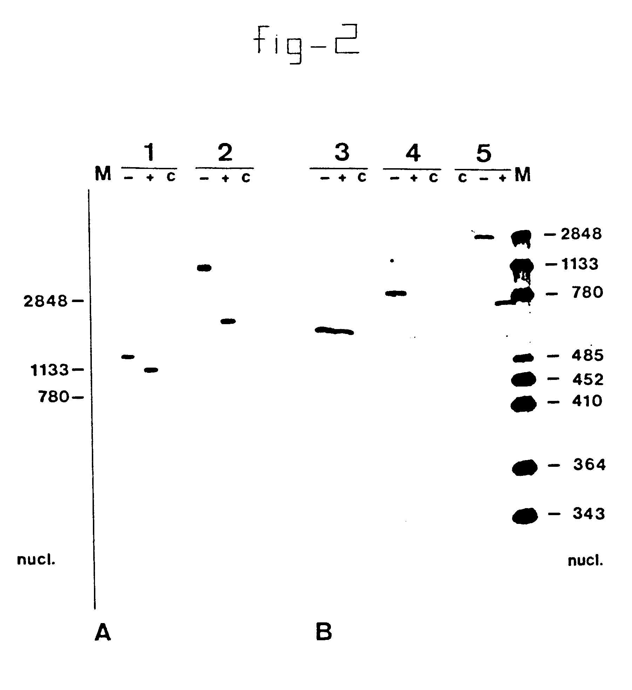

S1 nuclease protection experiments with human endothelial RNA were performed to proof that the various vWF cDNA inserts are fully complementary to vWF mRNA. The construction of the probes and the conditions used are described in the section Materials and methods. The results are shown in Figure 2. In all cases the length of the protected fragments is in accord with the predicted length of the different probes. From these data applicants conclude that the vWF cDNA inserts of, respectively, pvWF1330, pvWF1800 and pvWF2600 DNA are entirely complementary with vWF mRNA. The nucleotide sequence of the remaining cDNA inserts of plasmids pvWF2084 and pvWF2280 were shown before to correspond with the published sequence (Sadler, J.E., et al. (1985) Proc. Nucl. Acad. Sci. USA 82, 6394-6398). Consequently, the different, adjacent vWF cDNA sequences are genuine copies of vWF mRNA.

The vWF cDNA sequences that applicants have constructed span a length of about 8,900 bp. This length is consistent with the size of vWF mRNA, determined by Northern blot analysis of human endothelial (polyA) RNA (Verweij, C.L., et al. (1985) Nucleic Acids Research 13, 4699-4717, based on Dutch patent application 85.00961). A detailed description of the assembly of full length vWF cDNA is given in the section Materials and methods. The correct composition of the assembled, full length vWF cDNA was established by restriction enzyme analysis.

### Nucleotide sequence of full length vWF cDNA:

Nucleotide sequence analysis of vWF cDNA fragments was carried out both by the chemical degradation method (Maxam, A.M. et al., 1977) and by the dideoxy chain-termination procedure (Sanger, F., et al. (1977) Proc. Natl. Acad. Sci. USA 74, 5463-5467), according to the scheme outlined in Figure 1A. In Figure 3 the nucleotide sequence of about 8806 residues, extending from the 5' end of vWF mRNA, and the corresponding predicted amino acid sequence are presented. The remaining nucleotide sequence of the 3' part of vWF mRNA has been reported before (Sadler, J.E. et al., (1985) Proc. Natl. Acad. Sci USA 82, 6394-6398; Verweij, C.L. et al., (1985) Nucleic Acids Research 13, 4699-4717, based on Dutch patent application 85.00961). In general the overlapping part of our nucleotide sequence of vWF cDNA with that of Sadler, J.E. et al., (1985) Proc. Natl. Acad. Sci. USA 82, 6394-6398 reveals no differences. However, the first 12 nucleotides (corresponding with position 2217 till 2229) at the 5' terminus of vWF cDNA on phage lambda-HvWF1, constructed by those authors, are completely divergent from our sequence which has been established on three independent, overlapping cDNA inserts. Another discrepancy was observed at position 2309. Our analysis reveals a C residue, whereas Sadler, J.E. et al., (1985) Proc. Natl. Acad. Sci. USA 82, 6394-6398 report an A residue, resulting in, respectively, a proline and a histidine at that particular position. This difference might be due to a polymorphism in the vWF gene, although the proline residue has been established independently by automated amino acid sequence analysis of the mature vWF protein (Hessel, B. et al., (1984) Thrombosis Research 35, 637-651).

The total length spanned by the adjacent vWF cDNA sequences is 8,814 bp, excluding the polyA tail of vWF mRNA. The translation initiation site was assigned to the ATG codon indicated (position 1 till 4), being the first initiator codon downstream of the TAG nonsense codon (position - 79 till - 82), which is followed by an "open" translation reading frame of 8,439 nucleotides (deduced from our sequence data and those of Sadler, J.E. et al., (1985) Proc. Natl. Acad. Sci. USA 82, 6394-6398). This assignment is supported by the observation that the predicted 22 N-terminal amino acid residues displays the characteristic features of a signal peptide. The cleavage site for a signal peptidase with the highest probability is located between the glycine and alanine residues, respectively, at position 22 and 23 (Von Heijne, (1983) Eur. J. Biochem. 133, 17-21). The proposed translation initiation codon is preceded by an untranslated region of at least 230 nt.

A continuous vWF cDNA coding sequence of 8,439 bp potentially programs the synthesis of a polypeptide of 2,813 amino acid residues, with a calculated MW of 309 kD. To applicants' knowledge this represents the longest coding sequence determined to date. A computer aided search for (partial) homologous amino acid sequences with other proteins, contained within the NIH Protein Sequence Data Bank, did not reveal any major similarity with other proteins. Furthermore, it has been reported that mature vWF protein is a glycoprotein, containing approximately 15 % carbohydrate residues (Sodetz, J.M. et al., (1979) J. Biol. Chem. 254, 10754-10760). If, it is assumed that the carbohydrate moieties also contribute about 15 % by weight to calculated molecular weight of pro-vWF then the molecular weight of pro-vWF will amount to approximately 350 kD.

### Peculiarities of the amino acid sequence of Pre-vWF

A comparison of the predicted amino acid sequence (Figure 3) with the established N-terminal amino acid sequence of mature vWF protein (Hessel, B. et al., (1984) Thrombosis Research 35, 637-651) confirms our earlier assumption (Verweij, C.L. et al., (1985) Nucleic Acids Research 13, 4699-4717, based on Dutch patent application 85.00961) that the vWF precursor protein is considerably larger than the reported 260 kD glycoprotein (Wagner, D.D. et al., (1983) J. Biol. Chem. 258, 2065-2067). Alignment of the predicted amino acid sequence with the N-terminal sequence of the mature (225 kD) vWF protein shows that the nucleotide sequence which codes for mature vWF protein initiates at position 2290. This conclusion implicates that the vWF precursor protein is 763 amino acid residues larger than the mature protein. Obviously, this pro-sequence (calculated MW 81 kD) is removed by protein processing to yield the mature vWF glycoprotein with a molecular weight of about 225 kD.

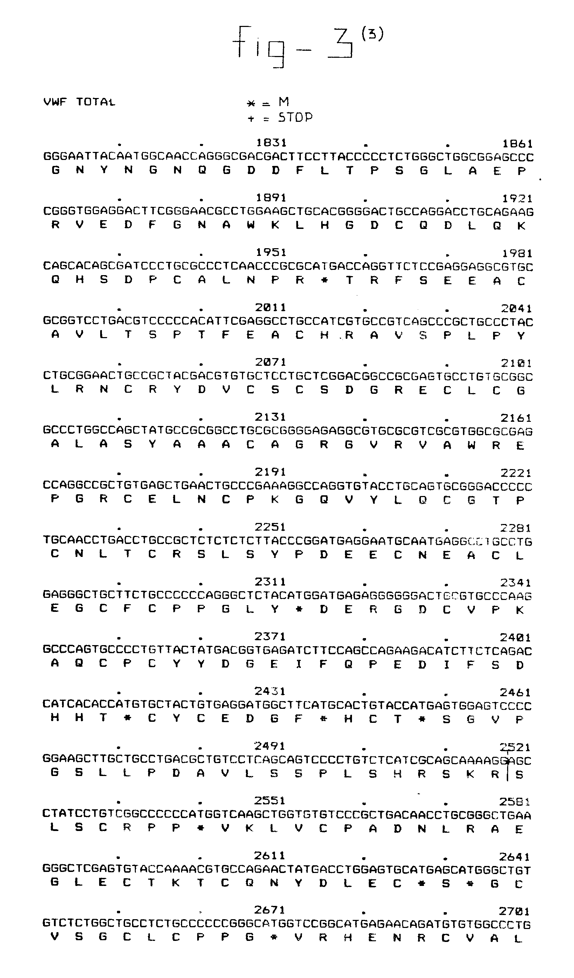

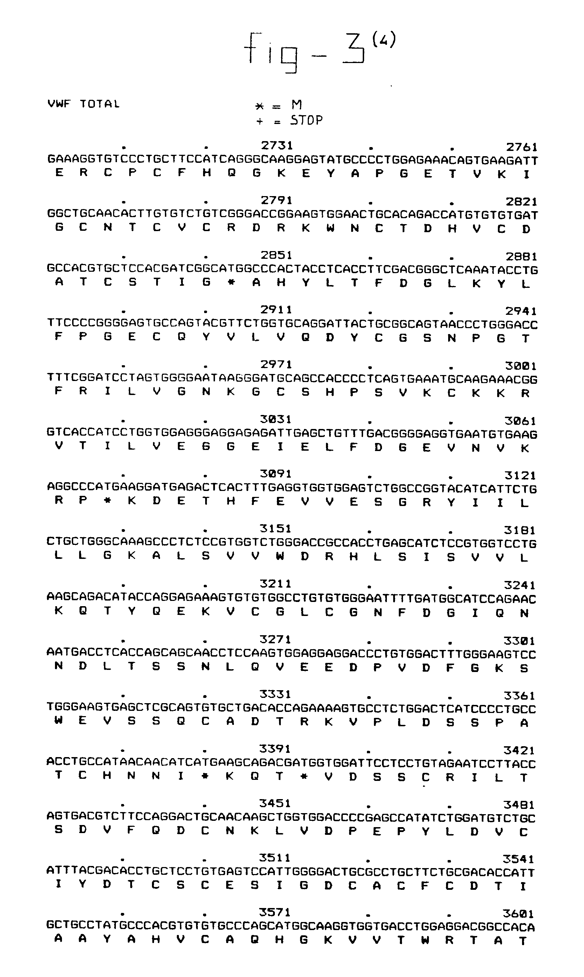

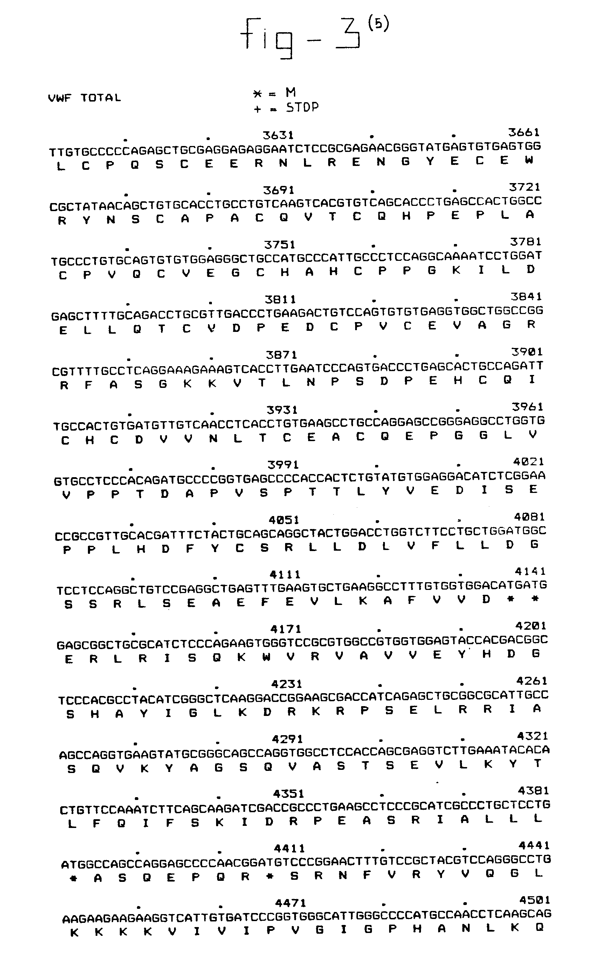

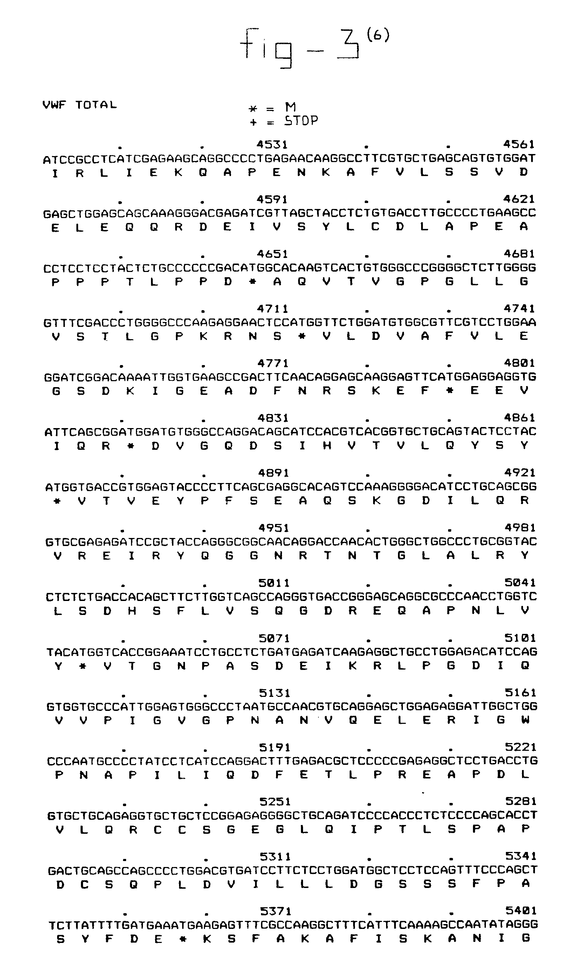

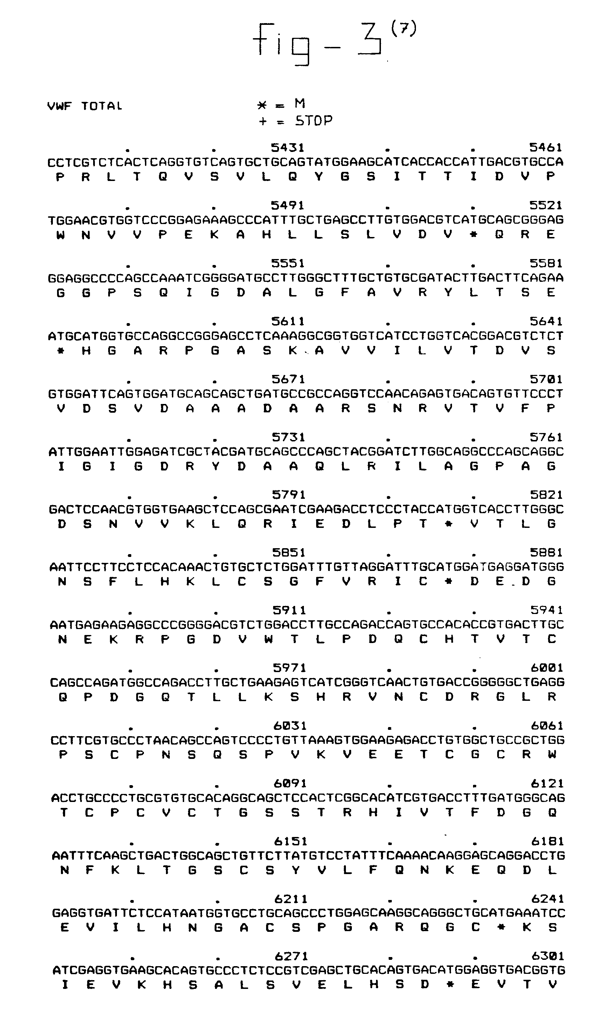

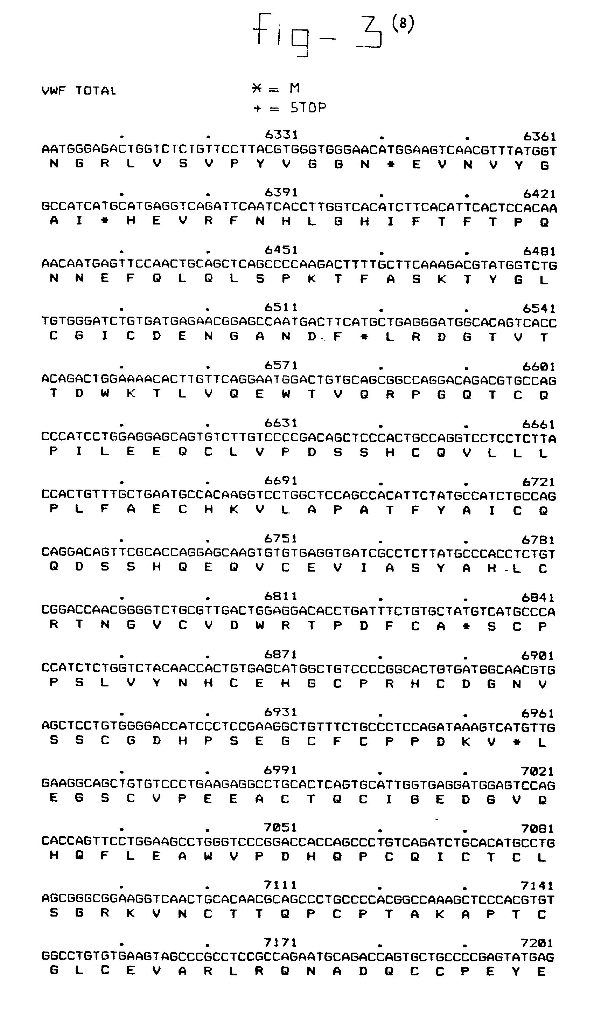

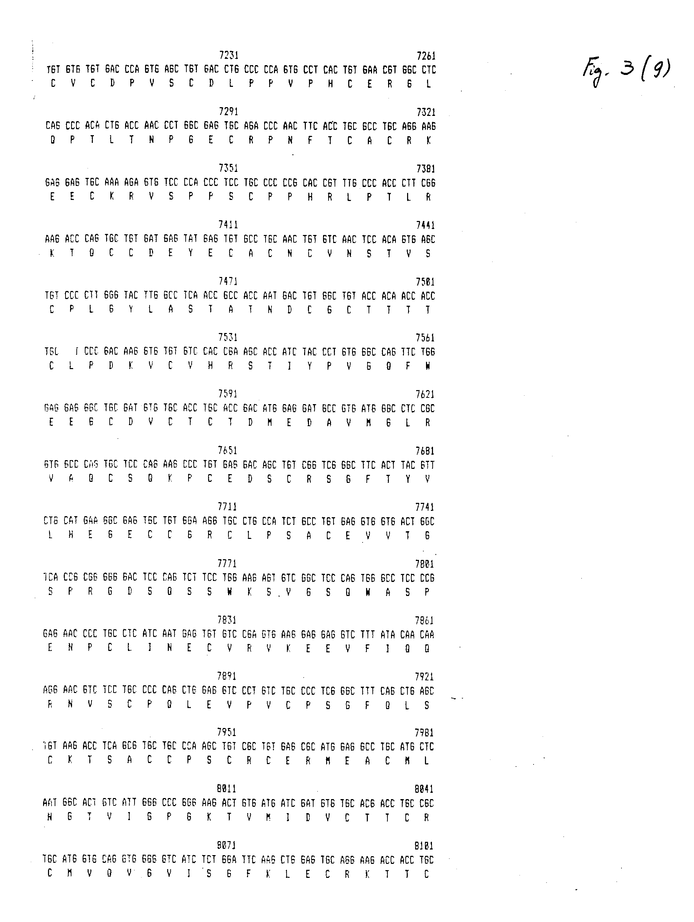

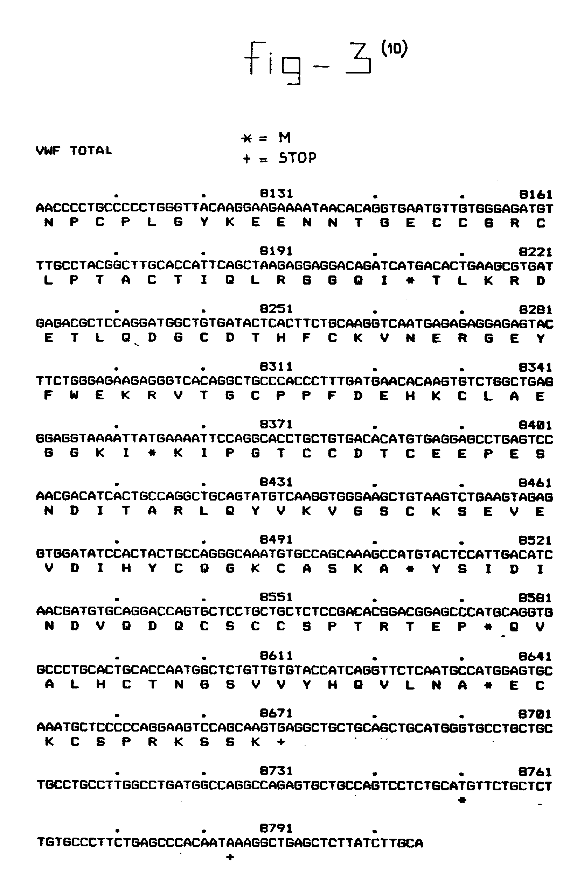

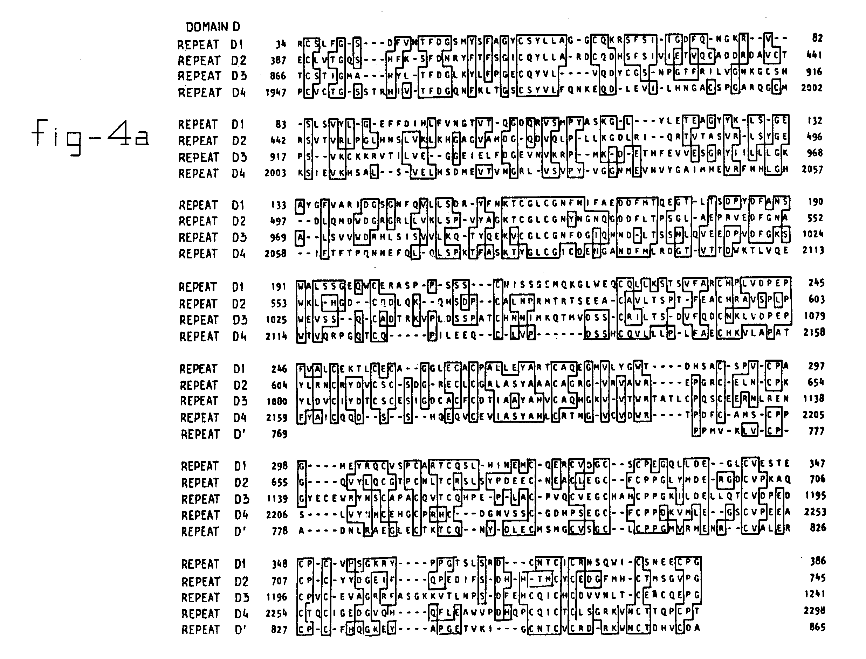

A homology matrix comparison of the amino acid sequence of the vWF precursor protein reveals a quadruplication of a domain (D1, D2, D3, D4) with a length of about 350 amino acid residues. Part of this domain (D', about 96 amino acids) appears to be present at the N-terminus of mature vWF. Figure 4A shows the alignment of these repetitive domains. A salient feature of the pro-sequence is that it largely consists of a duplication of the D domain (D1, D2). The repeats exhibit a significant conservation of the position of cysteine residues, indicative for a structural similarity of the repeats. Interestingly, the pro-sequence comprises an arginine-glycine-aspartic acid sequence ("RGD" sequence) at position 698 till 702. It has been shown that a tetrapeptide with the indicated amino acid sequence can compete with proteins, harboring a similar sequence, which are involved in cell attachment (Pierschbacher, M.D. et al., (1984) Nature 309, 30-33). It has been noticed that another RGD sequence is present within the C-terminal part of the mature vWF protein (Sadler, J.E. et al., (1985) Proc. Natl. Acad. Sci. USA 82, 6394-6398).

### In vitro translation of vWF mRNA

A full length vWF cDNA was assembled in order to demonstrate its coding capacity for a unglycosylated precursor protein with a Mw of about 300 kD. Full length vWF cDNA was inserted into plasmid pSP65. This plasmid contains the S. typhimurium bacteriophage SP6 promotor which allows in vitro "run off" transcription of cloned DNA sequences, specifically directed by SP6 RNA polymerase (Melton, D.A. et al., (1984) Nucl. Acids Research 12, 7035-7056). Such mRNA preparations can be efficiently translated, using a reticulocyte lysate.

Initially, plasmid pSP6330vWF (Figure 1B) was constructed, harboring a continuous, 6,331 bp vWF cDNA sequence. This plasmid contains the entire coding sequence for mature vWF and in addition a sequence coding for 18 amino acid residues from the C-terminal part of the pro-sequence. Initiation of protein synthesis, directed by RNA transcribed from pSP6330vWF DNA, should occur at the methionine codon 8 amino acids downstream of the N-terminus of mature vWF. Translation of the in vitro synthesized vWF mRNA will then yield an (unglycosylated) polypeptide with a calculated MW of 220 kD. Subsequently, pSP8800vWF (Figure 1B) was constructed harboring the complete coding sequence for pre-vWF. This plasmid will encode a protein with a calculated MW of 309,250 D. The plasmids pSP6330vWF and pSP8800vWF were linearized with, respectively, EcoRI and SalI and transcribed in vitro. The results of the in vitro translation of the various vWF mRNAs are given in Figure 5. The polypeptides encoded by pSP6330vWF DNA display a MW of about 200 kD. The discrepancy of this MW with the calculated MW (220 kD) is probably due to inaccuracy in the MW estimation of large proteins in these gels. The complete coding sequence of pSP8800vWF DNA is translated into a polypeptide with a MW substantially larger than 200 kD. To achieve a more accurate MW estimation for this extraordinarily long polypeptide, we produced partial, overlapping polypeptides derived from selected portions of full length vWF cDNA. To that end pSP8800vWF DNA was digested with BamHI and the transcript (2855 nt long) was translated. It should be noted that 405 nt at the 3' end of this transcript constitute the 5' terminus of the transcript generated from pSP6330vWF cleaved with EcoRI. Hence, an enumeration of the MWs of the polypeptides, mentioned above, should result in a MW of about 309 kD, after subtracting the common protein region. The protein, derived from the BamHI digested template, has an estimated MW of about 100 kD, while as shown before template pSP6330vWF cleaved with EcoRI yields a product of about 200 kD. Enumeration of these MWs and subtracting the common region (15 kD) results in an estimated MW of 285 kD which is in accord with the calculated MW of 309,250 D. Furthermore, translation of transcripts (1320 nt), derived from pSP8800vWF DNA digested with XhoI, reveals a polypeptide with a MW of 39 kD. This result is in agreement with the assignment of the translation initiation side at position 1 till 4.

From these data applicants conclude that they have constructed a full length vWF cDNA with a coding sequence of 8,439 bp which programs the synthesis of a precursor vWF protein consisting of 2,813 amino acid residues.

### DISCUSSION

This invention provides to the construction of a plasmid containing full length vWF cDNA. Nucleotide sequence analysis (this patent application; Verweij, C.L. et al., (1985) Nucleic Acids Research 13, 4699-4717, based on Dutch patent application 85.00961; Sadler, J.E. et al., (1985) Proc. Natl. Acad. Sci. USA 82, 6394-6398) revealed that the length of the assembled vWF cDNA, excluding cDNA derived from the polyA tail, amounts to 8,805 bp. This result is in agreement with the length of vWF mRNA (about 9,000 nt) as determined by Northern blot analysis of endothelial (polyA) RNA (Verweij, C.L. et al., (1985) Nucleic Acids Research 13, 4699-4717, based on Dutch patent application 85.00961). The entire coding sequence for a precursor vWF protein is 8,439 bp, corresponding to an unglycosylated polypeptide with a MW of about 309 kD. The translation initiation site for this protein could be assigned to the ATG codon at position 1 till 4 (see Figure 3). This assignment is in accord with the results of in vitro translation experiments with parts of full length vWF mRNA generated with the SP6 transcription system. The glycosylated protein, even after removal of a signal peptide, will be considerably larger than 300 kD. The MW attributed to the precursor glycoprotein for mature vWF has been reported to display a MW of 260 kD (Wagner, D.D. et al., (1983) Biol. Chem. 258, 2065-2067). The discrepancy with the MW that applicants assign to the precursor protein may be due to inaccuracy of MW estimations by SDS-polyacrylamide gelectrophoresis, inherent to large (glyco)proteins.

The sequence encoding mature vWF initiates at 2,286 bp downstream of the translation initiation codon, as inferred from an alignment of the established N-terminal amino acid sequence of mature vWF (Hessel, B. et al., (1984) Thrombosis Research 35, 637-651) with the predicted amino acid sequence (Figure 3). This 2,286 bp long prepro-sequence was shown to be able to encode a polypeptide with a calculated MW of 83 kD.
Consequently, the pro-vWF protein will be processed by a protease to yield two distinct polypeptides. It is conceivable that the specific cleavage between the arginine and serine residues at positions 763 and 764 occurs within the Weibel-Palade bodies of the endothelial cell, since in the presence of a protease inhibitor (PMSF) a complex can be detected within these organelles of mature vWF with another glycoprotein, designated von Willebrand Antigen II (vW AgII) (Montgomery, R.R. and Zimmerman T.S. 1978 J.Clin.Invest. 61, 1498-1507). Several arguments can be advanced which indicate that it is likely that the pro-sequence of the precursor vWF protein is identical to vW AgII.
i) the MW of the unglycosylated pro-sequence (83 kD) fits with the reported MW of the vW AgII glycoprotein of 98 kD.
ii) it has been demonstrated that both vWF and vW AgII are synthesized by cultured endothelial cells and that these proteins are simultaneously released upon stimulation with 1-desamino-β-D-arginine vasopressin (DDAVP) (McCarroll, D.R. et al, 1984 Blood 63, 532-535).
iii) it has been shown using immunofluorescence techniques that both proteins are localized in the perinuclear region and in the Weibel-Palade bodies (McCarroll, D.R. et al. (1985), J.Clin. Invest. 75, 1089-1095).
iv) both vWF and vW AgII are present in platelets and released together after platelet activation.
v) the levels of vWF and vW AgII protein are linearly associated in plasma and both proteins are deficient in plasma and platelets of a patient with severe von Willebrand's disease.
vi) as mentioned before a complex between vWF and vW AgII can be detected in the presence of a serine protease inhibitor (PMSF) and not in the absence of the inhibitor.

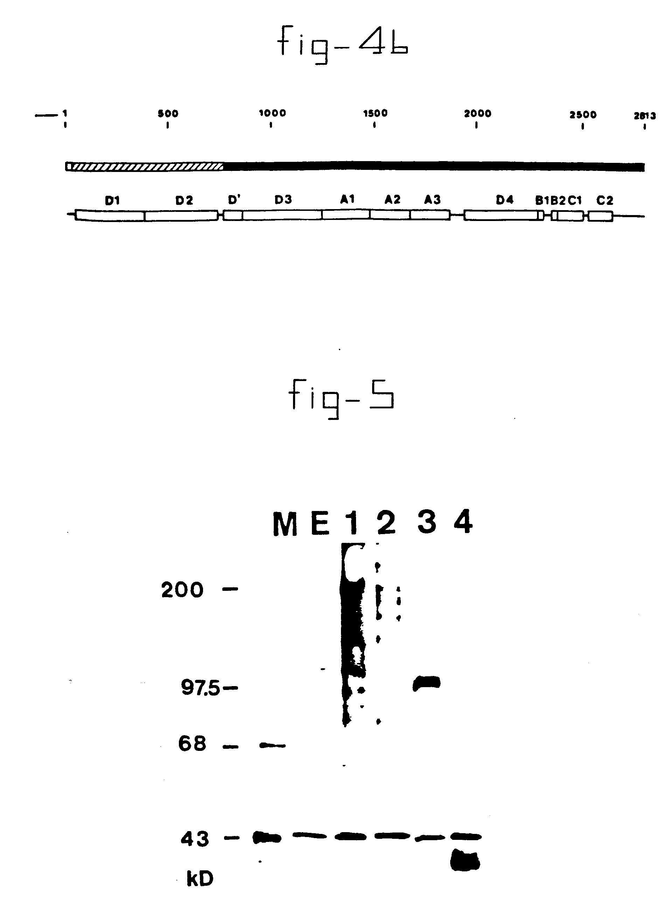

The predicted amino acid sequence of the pro-sequence displays a remarkable structure. It is composed of a duplicated segment of about 350 amino acid residues long. These two segments share 37 % amino acid homology. Furthermore, they exhibit a considerable conservation of similarly located cysteine residues, indicating that structural features have been maintained within these direct repeats. Two copies of this repeat within the pro-sequence are also present within mature vWF, whereas part of this repeat is present at the N-terminus of mature vWF. Internal homologous regions have also been reported by Sadler, J.E. et al, Proc. Natl.Acad.Sci.USA, 82, 6394-6398 (1985), two of which have been duplicated, while one is present in triplicate form (fig. 4B). These repeated sequences span a length of about 1070 amino acid residues within the mature vWF protein. The repeated structures that applicants have found are independent of the ones reported by Sadler, J.E. et al. Proc.Natl. Acad.Sci.USA 82, 6394-6398 (1985). From these data applicants conclude that about 90 % of the precursor vWF protein is constituted of repetitive regions, indicating that the precursor vWF gene has evolved from a series of duplicative events of at least four different regions.

The pro-sequence may participate in the formation of large multimers, composed of vWF dimers (Wagner, D.D. and Marder, V.J. 1984, J. of Cell Biology 99, 2123-2130; Lynch, D.E. et al (1983), The Journal of Biological Chemistry 258, 12757-12760). In this respect it may be relevant to note that pro-VWF is extremely rich in cysteines (8.1 %) which are mainly located in the N- and C-terminal parts of the protein. The cysteine content of the pro-sequence is even higher (8.6 %). It has been shown (Fowler, W.E. et al. J.Clin. Invest. 76, 1491-1500 (1985) that disulfide bonds at the C-termini of vWF are required to form rod shape domains, connecting two sub-units. Multimer formation should occur by joining of these dimers, probably again by disulfide bridges at their N-termini to create globular domains. Free sulfhydryl groups of the cysteine residues in the pro-sequence are probably a pre-requisite for the formation of multimers.

The presence of a "RGD(C)" amino acid sequence within the pro-sequence may be indicative for another function of this protein. It has been shown that an RGD containing region on proteins, such as fibronectin and vitronectin carry out a crucial role in the interaction with receptors on a cell surface (Pierschbacher, M.D. and Ruoslahti, E. (1984) Nature 309, 30-33); Pytela,R. et al. Proc.Nat. Acad.Sci.USA 82, 5766-5770). Those interactions are inhibited by RGD containing peptides. Interaction of mature vWF with activated platelets is also inhibited by RGD containing peptides, suggesting that this region on vWF is involved in platelet binding (Ginsburg, M., 1985 J.Biol.Chem. 260, 3931-3936); Haverstick, P.M. et al. 1985, Blood). Based on the presence of an RGD sequence both in mature vWF and the pro-sequence, which may be equivalent to vW AgII, and on a striking homology and a structural conservation between these two proteins, applicants assume that the pro-sequence might have a similar function as the mature vWF protein in a specific interaction with particular cell surface receptors.

### LEGENDS TO THE FIGURES

Fig. 1 Strategy for the construction of vWF cDNAs, the assembly of full length vWF cDNA and the determination of the nucleotide sequence.
   A) vWF mRNA is indicated by a bar; open area, signal peptide coding region; hatched area, pro-sequence coding region; solid area, mature vWF coding region.
      The oligonucleotides (20-mers) A (6901-6921), B (4819-4839) and C (2467-2487), which were used for primer-directed cDNA synthesis and/or as probe for hybridizations, are indicated by small bars. The 575 bp BGlII-BamHI and the 350 bp HindIII-XhoI fragments which were used as probes for colony-screening are indicated by open bars. Below the schematic representation of vWF mRNA the five partial, adjacent vWF cDNAs are given which were used for the assembly of full length vWF cDNA AND FOR NUCLEOTIDE SEQUENCING; The fragments I, II, III, IV and V, which were used in the S1 nuclease protection experiments, are shown above the vWF cBNA insert from which they were derived. The arrows indicate the nucleotide sequencing strategy. In the case of sequence analysis according to the procedure of Maxam and Gilbert (1977), the position of the radioactive labelling is given by a short vertical line at the end of an arrow. The slashes at the end of arrows mean that the end labelling was at a terminus, specified by vector DNA. Only restriction endonuclease sites which are relevant in this study are given. B, BamHI; Bg, BglII; E, EcoRI; H, HindIII; K, KpnI; M, Mst I; N, NarI; P, PvuII; S, SalI; Sc, SacI; X, XbaI; Xh, XhoI.
   B) Assembly of full length vWF cDNA. Plasmid pSP6330vWF contains a 6,331 bp vWF cDNA sequence, extending from the HindIII site (position 2235) till the SacI site (position 8562), subcloned in vector pSP64. Plasmid pSP8800vWF includes full length vWF cDNA, extending from the EcoRI site (see Panel A) till the SacI site (position 8562), subcloned in vector pSP65. Restriction endonuclease sites, delimiting the fragments used for the assembly of full length vWF cDNA, are indicated with an asterix. The EcoRI site at the 5' end of full length vWF cDNA originates from the EcoRI linker, used for the construction of pvWF1330 DNA. The sites for restriction enzymes, which were employed to linearize plasmid DNAs for in vitro "run off" transcription by SP6 RNA polymerase, are indicated by a dot. The SalI site in plasmid pSP8800vWF and the EcoRI site in plasmid pSP6330vWF are present in the polylinkers of the pSP-type vectors.
Fig.2 S1 nuclease protection analysis. Endothelial polyA+RNA was hybridized with [32P]-labelled probes, containing vWF cDNA sequences. The construction of the different probes and the conditions used are described in the section Experimental Procedures. The vWF cDNA segments, present in the probes, are shown in Fig. 1. Panel A shows the results after electrophoresis of the samples in a 0.8 % alkaline agarose gel. Panel B gives the results after electrophoresis in a 6 % polyacrylamide - 8 M urea gel. Lanes 1: hybridization with probe III, containing the 1,444 bp vWF cDNA fragment III (Fig. 1). Lanes 2: hybridization with probe V, containing the 2,400 bp vWF cDNA fragment V (Fig. 1). Lanes 3: hybridization with probe I which is equivalent to the 585 bp vWF cDNA fragment I (Fig. 1). Lanes 4: hybridization with probe IV, containing the 565 bp vWF cDNA fragment IV (Fig. 1). Lanes 5: hybridization with probe II, containing the 765 bp vWF cDNA fragment II (Fig. 1). Symbols: -, incubation of hybridized components in the absence of S1 nuclease; +, incubation of the hybridized components in the presence of S1 nuclease; c, incubation of the samples with S1 nuclease after hybridization in the absence of endothelial polyA+ RNA; M, single stranded DNA length markers.
Fig.3 Nucleotide sequence of 8806 bp of vWF cDNA, derived from the 5' terminus of vWF mRNA. The numbering starts at the putative ATG translation initiation codon. The predicted amino acid sequence is shown beneath the nucleotide sequence and are separately numbered, again starting at the putative methionine translation initiation codon. Potential N-linked glycosylation sites are underlined. The tripeptide arginine-glycine-aspartic acid is boxed.
Fig.4 Internal homology within the precursor for vWF.
   A) Alignment of the amino acid sequences of the four repeated domains D1, D2, D3, D4 and D'. The one-letter notation is used and the amino acids are numbered as indicated in Fig. 3. Residues which are identical among the four or five repeats are boxed.
   B) Schematic representation of internal homologous regions within pro-vWF. Indicated are the triplicated domain A (A1, A2 and A3) and two duplicated domains B (B1 and B2) and C (C1 and C2), as reported by Sadler et al. (1985), and the quadruplicated domain D (D1, D2, D3 and D4). The numeral position of these repeats are listed: A1 (residues 1242 till 1480), A2 (1480-1673), A3 (1673-1875), B1 (2296-2331), B2 (2375-2400), C1 (2400-2516), C2 (2544-2663), D1 (34-387), D2 (387-746), D3 (866-1242), D4 (1947-2299) and D' (769-866).
Fig. 5 In vitro translation of vWF mRNA.
   Capped vWF mRNA was prepared in vitro, using "run off" transcription with SP6 RNA polymerase, as described in the section Experimental Procedures. The RNA preparations were added to a reticulocyte lysate translation system, containing [35S]-methionine, and polypeptides were synthesized for 90 min. The polypeptides were fractionated on a 8 % SDS-polyacrylamide gel and then subjected to fluorography. M: MW marker proteins. E: endogeneously synthesized polypeptides (without added RNA). Lane 1: Polypeptides encoded by vWF mRNA transcribed from pSP6330vWF DNA, digested with EcoRI. Lane 2: Polypeptides encoded by vWF mRNA transcribed from pSPB8800vWF DNA, digested with SalI. Lane 3: polypeptides encoded by vWF mRNA transcribed from pSP8800vWF DNA, digested with BamHI. Lane 4: polypeptides encoded by vWF mRNA transcribed from pSP8800vWF DNA, digested with XhoI.

A sample of the recombinant DNA plasmid pSP8800vWF in strain E.coli DH 1 was deposited at the "Centraalbureau voor Schimmelcultures" in Baarn, The Netherlands, under number CBS 163.86 on March 26, 1986.

## Claims

1. Recombinant DNA, comprising a DNA fragment having a base sequence essentially as shown by nucleotides 229-8670 in figure 3 which codes for a full-length human von Willebrand factor precursor.

2. Recombinant DNA according to claim 1, further comprising a DNA fragment derived from a vector plasmid or phage.

3. A human or animal cell or microorganism which, as a result of genetic engineering, contains recombinant DNA as defined in claim 1 or 2.

4. A cell according to claim 3, which is capable of producing biologically active human von Willebrand factor.

5. A method for the preparation of biologically active human von Willebrand factor, comprising cultivating a cell as defined in claim 4 and isolating the biologically active human von Willebrand factor produced.

6. A method for the preparation of a pharmaceutical composition comprising biologically active human von Willebrand factor, comprising cultivating a cell as defined in claim 4, isolating and purifying the biologically active human von Willebrand factor produced, and formulating it together with a pharmaceutically acceptable carrier or excipient into a pharmaceutical composition.

## Patentansprüche

1. Rekombinante DNA, umfassend ein DNA-Fragment mit einer Basensequenz, wie sie im wesentlichen durch die Nukleotide 229-8670 in Fig. 3 gezeigt ist, welche einen Vorläufer des menschlichen von Willebrand-Faktors in voller Länge codiert.

2. Rekombinante DNA gemäss Anspruch 1, welche ferner ein von einem Vektorplasmid oder Phagen abgeleitetes DNA-Fragment enthält.

3. Menschliche oder tierische Zelle oder Mikroorganismus, welche gentechnologisch hergestellte rekombinante DNA, wie in Anspruch 1 oder 2 definiert, enthalten.

4. Zelle gemäss Anspruch 3, welche in der Lage ist, biologisch aktiven menschlichen von Willebrand-Faktor zu produzieren.

5. Verfahren zur Herstellung des biologisch aktiven menschlichen von Willebrand-Faktors, umfassend die Kultivierung einer Zelle, wie in Anspruch 4 definiert, und Isolierung des produzierten aktiven menschlichen von Willebrand-Faktors.

6. Verfahren zur Herstellung einer pharmazeutischen Zusammensetzung, die biologisch aktiven, menschlichen von Willebrand-Faktor umfasst, umfassend die Kultivierung einer Zelle, wie sie in Anspruch 4 definiert ist, Isolierung und Reinigung des produzierten, biologisch aktiven, menschlichen von Willebrand-Faktors und Formulierung desselben zusammen mit einem pharmazeutisch zulässigen Träger oder Corrigens zu einer pharmazeutischen Zusammensetzung.

## Revendications

1. ADN de recombinaison, comprenant un fragment d'ADN ayant une séquence de bases essentiellement comme montrée par les nucléotides 229-8670 sur la figure 3, qui code pour un précurseur du facteur de von Willebrand humain de longueur complète.

2. ADN de recombinaison selon la revendication 1, comprenant de plus un fragment d'ADN dérivé d'un phage ou d'un plasmide de vecteur.

3. Cellule animale ou humaine ou micro-organisme qui, en conséquence d'un processus d'ingénierie génétique, contient de l'ADN de recombinaison comme défini dans la revendication 1 ou 2.

4. Cellule selon la revendication 3, qui est capable de produire un facteur de von Willebrand humain biologiquement actif.

5. Procédé pour la préparation du facteur de von Willebrand humain biologiquement actif, comprenant la culture d'une cellule comme définie dans la revendication 4 et l'isolation du facteur de von Willebrand humain biologiquement actif ainsi produit.

6. Procédé pour la préparation d'une composition pharmaceutique comprenant le facteur de von Willebrand humain biologiquement actif, comprenant la culture d'une cellule comme définie dans la revendication 4, l'isolation et la purification du facteur de von Willebrand humain biologiquement actif produit, et sa formulation en une composition pharmaceutique avec un support ou excipient pharmaceutiquement acceptable.
